# EUROPEAN PATENT APPLICATION

(11) **EP 0 897 927 A1**
(43) Date of publication of application: **24.02.1999**
(21) Application number: 98202642.9
(22) Date of filing: 05.08.1998
(51) Int. Cl.: C07J 1/00, A61K 31/565

(54) **Novel crystalline form of the progestagen - (17alpha) 17-hydroxy-11-methylene-19-nor-pregna-4,15-dien-2 0-yn-3-one(Org 30659)**

(30) Priority: 11.08.1997 EP 97202472
(71) Applicant: Akzo Nobel N.V., 6824 BM Arnhem (NL)
(72) Inventor: Booy, Cornelis Johannus, 5345 VC Oss (NL); Brands, Franciscus Theodorus Leonardus, 5368 AZ Haren (NL); de Wildt, Wilhelmus Petrus Hubertus, 6604 EG Wychen (NL); van der Schans, Maria Jacoba Adriana, 5161 EZ Sprang-Capelle (NL)
(74) Representative: Kraak, Hajo

(57) **Abstract**

The disclosed invention resides in the polymorphism found with the progestagenic steroidal compound (17α)-17-hydroxy-11-methylene-19-norpregna-4,15-diene-20-yn-3-one. More particularly, the invention resides in crystalline forms of said compound having unexpectedly favourable thermodynamic stability. These crystalline compounds, which can be suitably described with reference to spectral data, can advantageously be employed as an active ingredient in solid pharmaceutical compositions. The crystalline compounds can be used in oral contraceptives and in hormone replacement therapy (HRT).

## Description

The invention pertains to crystalline (17α)-17-hydroxy-11-methylene-19-norpregna-4,15-diene-20-yn-3-one, hereinafter referred to as Org 30659. Org 30659 is a progestational steroid with high progestagenic activity and with low estrogenic and low androgenic activity. Org 30659 is suitable for being utilised in hormone replacement therapy (HRT) and in contraception.

Org 30659 as a crystalline chemical compound is known from EP 210 678. A drawback to the Org 30659 produced therein is that it is not obtained in one single, reproducible crystalline form. Further, the thermodynamic stability of the crystals obtainable needs to be improved.

Another background disclosure on Org 30659 is WO 96/09056, which pertains to a process of making dosage units comprising e.g. Org 30659 in a relatively low amount, which process involves wet granulation. WO 96/09056 refers to Org 30569 as a known compound without specifically teaching its crystallinity or the selection of a particular preparation method.

The above drawback was found to be associated with crystalline Org 30659 being polymorphous, i.e. it can occur in more than one crystalline form. As frequently recognised nowadays, for pharmaceutical compounds, polymorphism in itself can be disadvantageous. The possibility of one crystalline form converting to the other, depending on the circumstances during preparation, storage, or use of a pharmaceutical composition comprising a crystalline form of the polymorphous compound as the pharmaceutically active ingredient, makes the stability and bio-availability of the active ingredient less well predictable. In order for the amounts in pharmaceutical compositions and dosages administered during therapy to be determined in sufficiently exact manner, it is important to have certainty beforehand on the bio-availability of the compound. Such product consistency is of importance when the product is processed into and used as a pharmaceutical composition in general and, all the more, when the composition is of a sustained release type.

In a general sense, the invention resides therein that new crystalline forms of Org 30659 were found to occur, which forms are characterised by having a thermodynamic stability (ΔH) of about 70 mJ/mg or higher. By providing Org 30659 in any one of these forms, it is rendered into a consistent product, having a predictable presence and bio-availability. The preferred of these forms can also be characterised by their having a melting point of at least 160°C. Hence, surprisingly, crystalline forms of Org 30659 were found which, apart from being obtainable in a crystalline pure form, are more stable than that obtainable in accordance with the disclosure of EP 210 678. Employing a crystalline compound of the invention in further processing Org 30659 crystals, and ensuring that a crystalline compound of the invention constitutes the crystalline Org 30659 introduced into a pharmaceutical composition, will make for pharmaceutical compositions of Org 30659 which enjoy a high consistency and stability, and which lead to a high predictability of the available dose of the crystalline compound.

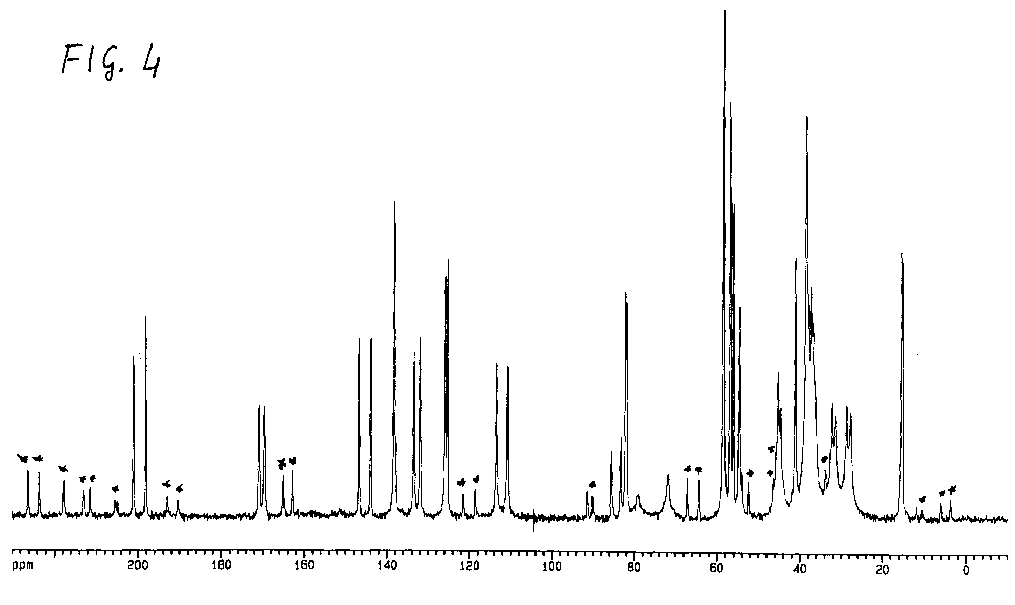

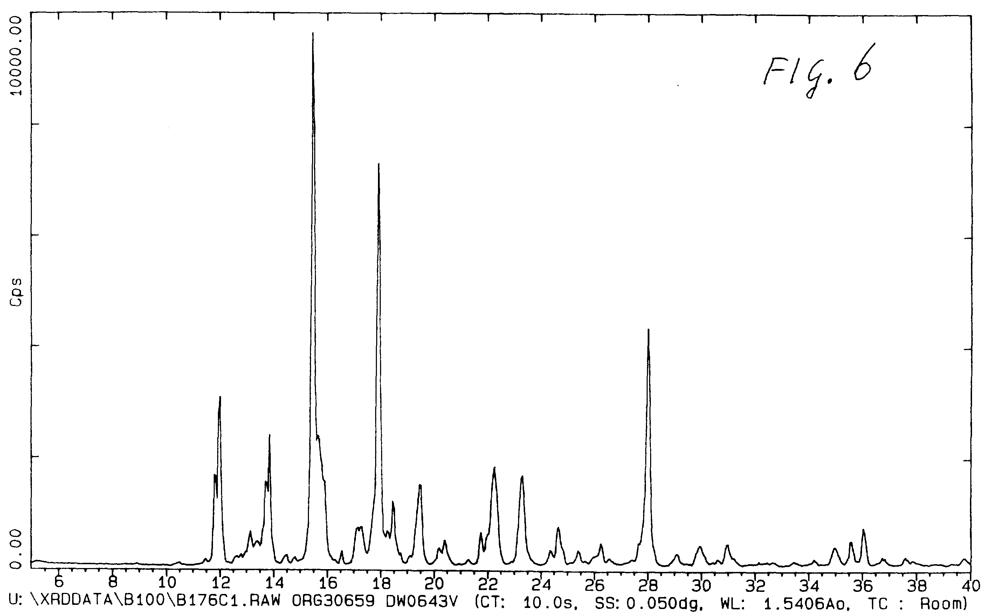

The invention, in a preferred embodiment, also resides in crystalline Org 30659 having one specific crystalline form, which is characterised by the spectra of figures 4 and 6, as discussed hereinafter. This particular crystalline form of Org 30659 exhibits an unexpectedly high thermodynamic stability, as shown int.al. by an enthalpy value ΔH of about 80 mJ/mg or higher according to DSC (differential scanning calorimetry) measurements. In view of the desired consistency, stability, and predictability, this form is preferred.

For the preparation of Org 30659 as a chemical compound, reference is made to EP 210 678. The crystalline form obtained was found to depend on the choice of a medium for (re)crystallisation.

Thus, if the crude product obtained in accordance with EP 210 678 is crystallised from e.g. a mixture of ethyl acetate and n-heptane, Org 30659 is obtained in the pure crystalline form satisfying the spectra of figures 4 and 6, hereinafter referred to as form (A). The crystalline Org 30659 having form (A) displays a ΔH of 84 mJ/mg and has a melting point of 164 °C (both as determined by DSC). At temperatures below 155°C, including subjection to boiling water, this form (A) is the most stable one. Several ratios of ethyl acetate to heptane can be used. It is preferred for the heptane to be in excess. As a co-solvent hexane may be substituted for heptane.

In this respect, the invention also pertains to a process for the preparation of crystalline Org 30659 comprising the steps of synthesizing Org 30659 and crystallising it from a solvent, with or without seeding. In general such this is known from EP 210 678, but has now been found to have the drawback that it does not reproducibly result in one single crystalline form of Org 30659. According to the process of the invention this drawback is obviated by selecting as the solvent the above-identified mixture of ethyl acetate and n-heptane.

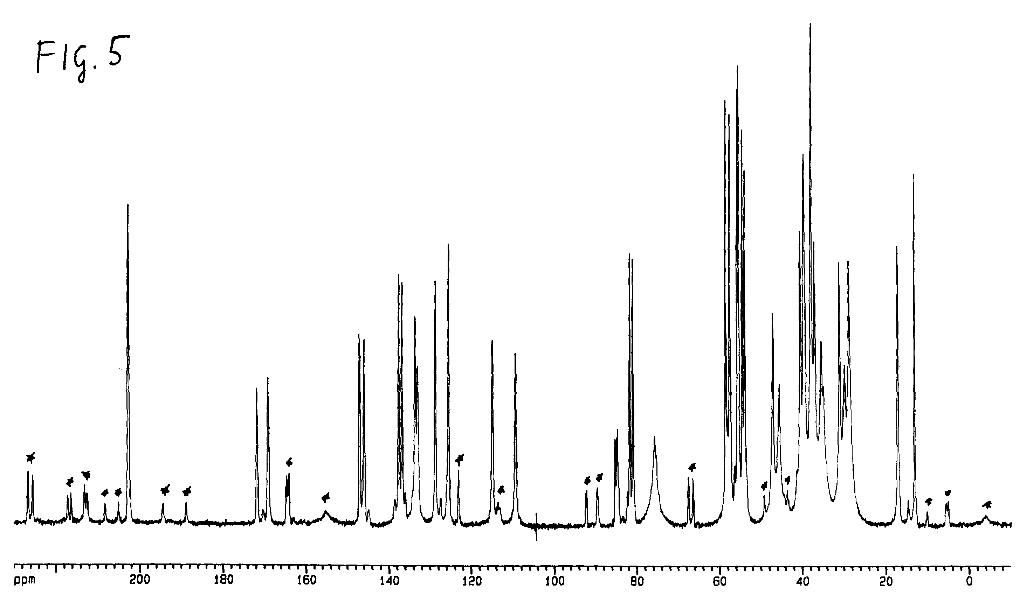

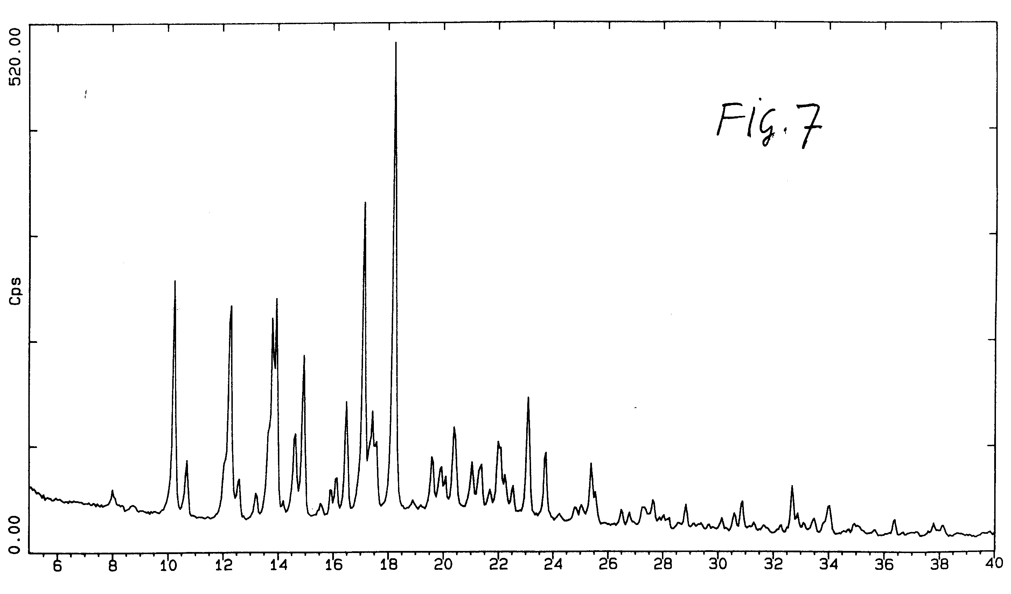

A further process according to the invention is heating the crystals obtained as above to approximately 162°C. This makes for conversion to another crystalline form, viz. one satisfying the spectra of figures 5 and 7, hereinafter referred to as form (B). The Org 30659 in this crystalline form (B) has a lower enthalpy, viz. a ΔH of 70 mJ/mg, but a higher melting point of 166.7°C (both data obtained from DSC). This form B can also be obtained using so-called anti-solvent crystallisation conditions. This is a principle known in the art, which can be applied by the person of ordinary skill in the art without undue experimentation.

It should be noted that the above relates to two out of more possibilities to obtain crystalline forms of Org 30659 which have an enthalpy ΔH of 70 mJ/mg or higher and a melting point of over 160°C. It is not to be excluded that, by virtue of the recognition according to the invention that crystalline Org 30659 can be produced in a more stable form than was known, the person of ordinary skill in the art will be able to find other stable forms than those denoted (A) and (B) for which the above clear guidelines are given. Such other forms of Org 30659, i.e. those forms having a melting point of at least 160°C, an enthalpy as measured by DSC of over 70 mJ/mg, or both, expressly are in accordance with the present invention. E.g., yet another process-possibility is to crystallise the compound from a solvate-forming solvent such as toluene, and subsequently produce a transformation of the solvate crystals by subjecting them (neat or in suspension) to one or more heating steps to remove the solvent from the solvate.

The invention also pertains to a pharmaceutical composition comprising Org 30659 and a pharmaceutically acceptable carrier. Preferably, Org 30659 is present in a pharmaceutical composition in any one of the above crystalline forms. For the best extent of knowing beforehand which form will be contained in a pharmaceutical formulation, and for being sure that this form has sufficient stability for it to be unchanged, it is most preferred to select form (A) as the active, crystalline compound of a solid pharmaceutical formulation.

A pharmaceutical formulation according to the invention will generally take the form of a dosage unit such as a tablet or a capsule, but other solid or dry pharmaceutical preparations are included. Methods for making such dosage units are well known. For example in the standard English language text Gennaro et al., Remington's Pharmaceutical Sciences, (18th ed., Mack Publishing Company, 1990, see especially Part 8: Pharmaceutical Preparations and Their Manufacture), methods of making tablets, capsules and pills and their respective components are described. Daily doses of Org 30659 typically are in a range of from a few micrograms (as few as possible, but by general preference at least 7,5 µg) to 240 µg. Also for controlled (slow) release preparations of crystalline Org 30659 it is preferred that form A be selected.

Org 30659, being in a crystalline form selected in accordance with the present invention, can be processed in accordance with the disclosure of WO 96/09056, or any other suitable dry or wet processing method available in the art.

In the pharmaceutical compositions of the invention, Org 30659 can be present as the only pharmaceutically active ingredient, or combined with other pharmaceutically active compounds, e.g. an estrogenic compound such as ethinyl estradiol, estradiol, or esters thereof. Org 30659 itself preferably is used in one pure, crystalline form, i.e. without any other crystalline form being present. In the art of making pharmaceutical compositions of compounds exhibiting polymorphicity, the presence of 90% or more of a single crystalline form is considered to be an acceptable crystalline purity. It should be noted that such a pure crystalline form is not obtained in EP 210 678.

The pharmaceutical compositions of the invention may further comprise excipients, additives, adjuvants, and other conventional auxiliaries.

The invention furthermore pertains to the use of crystalline Org 30659 having a crystalline form as described hereinbefore, for the preparation of an oral contraceptive as well as for the preparation of a medicament for HRT. The advantage of using a form according to the invention in preparing such medicinal agents resides, int.al., in the consistency of the physical state of the compound. The better the physical stability, the more remote the chance that a compound will transform from one crystalline form into the other. By avoiding crystalline transformations, as a result of using a crystalline form according to the invention, any adverse influences of such transformations on the bio-availability of the active compound are avoided as well. In this respect form A according to the invention is preferred.

The invention is further explained with reference to the following, unlimitative description of Methods, Examples and Figures.

### METHODS

### Differential Scanning Calorimetry (DSC)

DSC curves were recorded using a Seiko DSC-120 instrument. Closed aluminium pans having a volume of 5µl were used in combination with a nitrogen flow of 50 ml/min. The heating rate was 5.0°C/min.

### NMR Spectroscopy

Solid state ¹³C NMR recorded using a Bruker MSL-400 spectrometer with a spectral frequency of 100.6 MHz, a spinning rate of approximately 10 KHz, an acquisition time of 65 ms, and a 90° pulse of 4 µs or a Bruker DRX-400 operating at 100.6 Mhz, a 90° pulse of 5.0 µs, an acquisition time of 102 ms and a spinning rate of 8 KHz.

### X-Ray Powder Diffraction (XRPD)

The XRPD patterns were recorded using a Philips PW1050 reflection-diffractometer with Cu-Kα radiation, the generator settings being 40 kV, 40 mA. Splits: 0.5°, 0.2 mm, 0.5°. In the case of (A): measuring range: 2θ=2-50°; stepsize: 0.05°; time per step: 10 s., in the case of (B) a Philips PW1050 transmission-diffractometer was used: measuring range 2θ=4-50°; stepsize: 0.05°; time per step 60 s.

### EXAMPLES

### Example 1 _

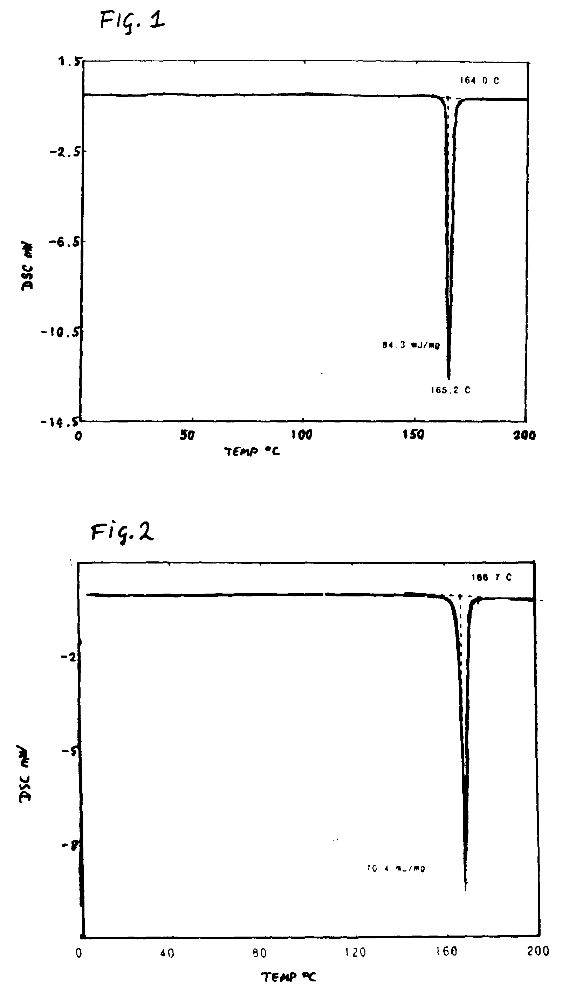

100 grams of Org 30659 are dissolved in 300 ml ethyl acetate at reflux temperature. At this temperature 300 ml of heptane are added in 15 minutes. The solution is cooled to 70 °C and at this temperature the mixture is seeded with 0.2 gram crystalline form A to initiate crystallisation The temperature is raised again to reflux temperature and 1200ml of heptane are introduced during 30 minutes. Stirring at reflux temperature is continued for 2 hours. After that the suspension is allowed to cool to room temperature and subsequently stirred at -15 °C for two hours. The crystals are filtered off washed with cold heptane and dried *in vacuo* at 50 °C. Obtained are 91.4 g of Org 30659 in the crystalline form denoted (A), and having the various spectra depicted in Figures 1, 4, and 6.

### Example 2 _

100 grams of Org 30659 are dissolved in 300 ml ethyl acetate at reflux temperature. This warm solution is added over a period of 45 minutes to a suspension of 1500 ml of heptane and 0.2 grain of crystalline form B at room temperature. The suspension is stirred for additional 1 hour at room temperature and 2 hours at - 15 °C. The crystals are filtered off washed with cold heptane and dried *in vacuo* at 50 °C . Obtained are 88.7 g of Org 30659 in the crystalline form denoted (B), and having the various spectra depicted in Figures 2, 5, and 7.

### Example 3 _

100 grams of Org 30659 are dissolved in 450 ml of toluene at reflux temperature. Subsequently the solution is allowed to cool to room temperature and is stored overnight at -15°C. The resulting toluene solvate crystals are filtered and dried *in vacuo*. The full yield of 124 g is then heated at 60-65°C *in vacuo*, to form 95 grams of pure Org 30659 in crystal form X, as determined by solid state ¹³C-NMR analysis. Thereupon, 10 grams of Org 30659 in form X are heated at 140°C during 90 minutes *in vacuo*. Obtained are 10 grams of Org 30659 in crystalline form C according to the invention, having a melting point of 158.7°C and an enthalpy of 71.9 mJ/mg.

### FIGURES

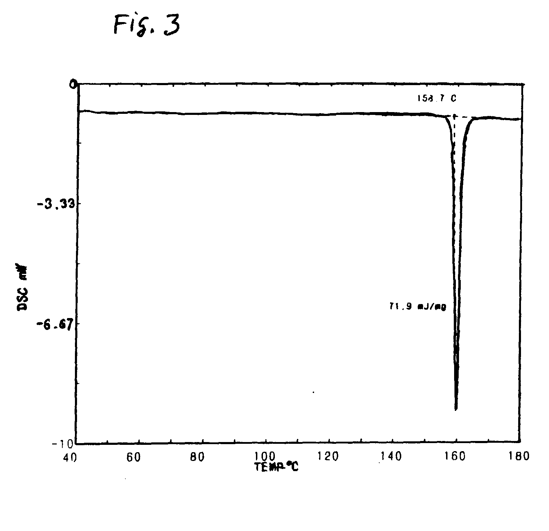

*Figures 1-3*
   DSC curves of crystalline forms of Org 30659. The horizontal axis represents the temperature in °C (heating), the vertical axis represents heat in µW or mW.
   FIG.1 is the DSC curve for crystalline form (A) of the invention, showing a peak at the melting temperature of 164.0 °C, from which the enthalpy ΔH is determined to be 84.3 mJ/mg.
   FIG.2 is the DSC curve for crystalline form (B) of the invention, showing a peak at the melting temperature of 166.7°C, the enthalpy ΔH being determined as 70.4 mJ/mg.
   FIG.3 is the DSC curve for crystalline form (C) of the invention, showing a peak at the melting temperature of 158.7°C, the enthalpy ΔH being determined as 71.9 mJ/mg.
*Figures 4-5*
   Solid state ¹³C NMR spectra of crystalline forms of Org 30659. The horizontal axis conventionally represents the chemical shift (in ppm) relative to adamantane as a standard (38.56 ppm), The vertical axis indicates the peak-intensity.
   FIG.4 is the NMR spectrum of form (A) of the invention.
   FIG.5 is the NMR spectrum of form (B) of the invention.
   The most significant chemical shifts (in ppm) that can be determined are listed in Table 1 below. Also given in Table 1, are the chemical shifts allocated to two crystalline forms identified in the case of Org 30659 as produced in EP 210 678.
*Figures 6-7*
   XRPD spectra of crystalline forms of Org 30659. The horizontal axis conventionally represents the reflections, the vertical axis indicates the peak-intensity.
   FIG. 6 is the XRPD spectrum of form (A) of the invention. The most significant reflections that can be determined are listed in Table 2 below.
   FIG. 7 is the XRPD spectrum of form (B) of the invention. For the reflections reference is made to Table 2.
   Also given in Table 2, are the reflections allocated to two crystalline forms identified in the case of Org 30659 as produced in EP 210 678.

**TABLE 1**

| ¹³C NMR chemical shifts | | | | | |
|---|---|---|---|---|---|
| Atom number | Crystalline form | | | | |
| | EP 210 678 (X) | EP 210 678 (Y) | A | B | C |
| 3 | 202.7 | 200.3 | 198.2 | 202.7 | 201.4 |
| | | | 201.0 | | |
| 4 | 125.5 | 124.6 | 125.3 | 125.5 | 126.5 |
| | | | 125.9 | 128.7 | |
| 5 | 172.7 | 170.4 | 169.7 | 169.2 | 170.4 |
| | | | 171.0 | 171.9 | |
| 11 | 144.4 | 144.4 | 144.0 | 146.0 | 146.7 |
| | | | 146.7 | 147.1 | |
| 11' | 113.2 | 109.8 | 110.8 | 109.3 | 112.1 |
| | | | 113.4 | 114.9 | |
| 15 | 132.1 | 127.8 | 131.9 | 133.0 | 130.6 |
| | | | 133.5 | 133.6 | |
| 16 | 137.8 | 137.7 | 138.2 | 136.8 | 139.8 |
| | | | | 137.6 | |
| 17 | 80.6 | 78.4 | 81.9 | 81.0 | 80.7 |
| | | | 82.2 | 81.7 | |
| 18 | 18.8 | 12.8 | 15.3 | 13.1 | 14.8 |
| | | | 15.6 | 17.1 | |
| 20 | 85.2 | 82.0 | 83.3 | 84.8 | 85.2 |
| | | | 85.6 | 85.3 | |
| 21 | 76.2 | 73.1 | 71.8 | 75.7 | 75.2 |
| | | | 79.0 | | |

**TABLE 2**

| XRPD reflections | | | | | | | | | |
|---|---|---|---|---|---|---|---|---|---|
| **EP 210678 X** | | **EP 210678 Y** | | **A** | | **B** | | **C** | |
| 2θ | r.i. % | 2θ | r.i. % | 2θ | r.i. % | 2θ | r.i. % | 2θ | r.i. % |
| 9.66 | 34 | 9.65 | 21 | 11.78 | 18 | 7.98 | 12 | 11.12 | 17 |
| 10.91 | 24 | 10.89 | 16 | 11.94 | 32 | 10.15 | 53 | 11.46 | 11 |
| 12.27 | 10 | 12.29 | 13 | 13.18 | 7 | 10.65 | 18 | 13.09 | 42 |
| 13.28 | 20 | 12.39 | 13 | 13.39 | 5 | 12.00 | 17 | 13.53 | 84 |
| 15.08 | 94 | 13.26 | 20 | 13.66 | 8 | 12.23 | 48 | 15.06 | 83 |
| 15.65 | 49 | 13.52 | 34 | 13.72 | 16 | 12.56 | 14 | 17.22 | 100 |
| 17.39 | 100 | 13.80 | 18 | 13.85 | 25 | 13.15 | 11 | 17.61 | 52 |
| 17.99 | 10 | 15.15 | 100 | 15.45 | 100 | 13.58 | 23 | 19.41 | 54 |
| 18.68 | 58 | 15.50 | 25 | 15.68 | 24 | 13.77 | 46 | 20.12 | 19 |
| 20.30 | 11 | 15.65 | 35 | 17.13 | 8 | 13.83 | 50 | 20.61 | 10 |
| 20.84 | 23 | 16.16 | 25 | 17.27 | 8 | 14.59 | 23 | 21.38 | 40 |
| 21.81 | 97 | 16.79 | 31 | 17.93 | 76 | 14.84 | 39 | 21.53 | 31 |
| 22.19 | 23 | 17.01 | 13 | 18.26 | 7 | 15.87 | 12 | 22.62 | 11 |
| 24.78 | 20 | 17.40 | 92 | 18.42 | 13 | 16.09 | 14 | 23.12 | 7 |
| 25.33 | 13 | 18.71 | 42 | 19.44 | 16 | 16.42 | 29 | 23.87 | 52 |
| 26.48 | 21 | 19.49 | 42 | 20.36 | 6 | 17.04 | 69 | 24.17 | 22 |
| 26.75 | 12 | 21.02 | 29 | 21.75 | 7 | 17.40 | 28 | 24.68 | 19 |
| 27.75 | 25 | 21.84 | 87 | 22.24 | 19 | 17.55 | 21 | 26.73 | 26 |
| 29.75 | 19 | 22.35 | 30 | 23.26 | 18 | 18.16 | 100 | 27.48 | 17 |
| 31.80 | 18 | 22.56 | 10 | 24.67 | 8 | 19.57 | 19 | 30.03 | 20 |
| 32.31 | 22 | 23.52 | 14 | 26.24 | 5 | 19.88 | 17 | 30.39 | 14 |
| 33.11 | 18 | 24.32 | 40 | 27.63 | 5 | 20.05 | 15 | 30.67 | 17 |
| 33.50 | 12 | 24.77 | 15 | 28.01 | 45 | 20.38 | 24 | 30.89 | 21 |
| 34.95 | 13 | 26.51 | 15 | 29.95 | 5 | 20.97 | 17 | 31.59 | 27 |
| 35.16 | 10 | 26.75 | 12 | 30.99 | 5 | 21.29 | 17 | 34.44 | 12 |
| | | 27.80 | 20 | 34.99 | 4 | 21.97 | 21 | 34.94 | 37 |
| | | 28.13 | 13 | 35.59 | 5.51 | 22.06 | 20 | | |
| | | 29.78 | 15 | 36.09 | 8 | 22.21 | 15 | | |
| | | 31.87 | 10 | | | 22.50 | 13 | | |
| | | 32.34 | 10 | | | 23.03 | 30 | | |
| | | 33.18 | 13 | | | 23.65 | 19 | | |
| | | 35.24 | 10 | | | 25.40 | 17 | | |
| | | | | | | 32.66 | 13 | | |

## Claims

1. Crystalline (17α)-17-hydroxy-11-methylene-19-norpregna-4,15-diene-20-yn-3-one (Org 30659), characterised by having an enthalpy ΔH of at least 70 mJ/mg as measured by the DSC technique referred to in the description.

2. Org 30659 according to claim 1, characterised in that it has a melting point of at least 160°C.

3. Org 30659 in a crystalline form, characterised in that the crystalline form is represented by the solid state ¹³C NMR spectrum of Figure 4.

4. Org 30659 in a crystalline form, characterised in that the crystalline form is represented by the X-Ray Powder Diffraction pattern, of Figure 6.

5. A solid pharmaceutical composition comprising a pharmaceutically acceptable carrier and at least one pharmaceutically active ingredient, the pharmaceutically active ingredient being the compound Org 30659 in a crystalline form, characterised in that the crystalline form is selected from the group consisting of crystalline forms of said compound having a melting point of at least 160°C as measured by the DSC technique referred to in the description.

6. A solid pharmaceutical composition comprising a pharmaceutically acceptable carrier and at least one pharmaceutically active ingredient, the pharmaceutically active ingredient being the compound Org 30659 in a crystalline form, characterised in that the crystalline form is selected from the group consisting of crystalline forms of said compound having an enthalpy of over 80 mJ/mg as measured by the DSC technique referred to in the description.

7. A solid pharmaceutical composition according to claim 6, characterised in that the active ingredient is the compound of claim 3.

8. A solid pharmaceutical composition according to claim 6, characterised in that the active ingredient is the compound of claim 4.

9. A solid pharmaceutical composition according to any one of the claims 6-8, characterised in that the composition is a preparation for controlled, continuous release of Org 30659.

10. A solid pharmaceutical composition comprising Org 30659 and a pharmaceutically acceptable carrier, characterised in that Org 30659 is present in the composition in one single crystalline form.

11. A process for the preparation of crystalline Org 30659 comprising the steps of synthesizing Org 30659 and crystallising it from a solvent, characterised in that the solvent is a mixture of ethyl acetate and n-heptane.

12. The use of crystalline Org 30659 having a crystalline form according to any one of the claims 1-4 for the preparation of an oral contraceptive.

13. The use of crystalline Org 30659 having a crystalline form according to any one of the claims 1-4 for the preparation of a medicament for HRT (hormone replacement therapy).
